(19) Europäisches Patentamt
European Patent Office
Office européen des brevets

(11) Veröffentlichungsnummer: **0 553 673 A1**

# (12) EUROPÄISCHE PATENTANMELDUNG

(21) Anmeldenummer: **93100650.6**

(22) Anmeldetag: **18.01.93**

(51) Int. Cl.5: **C07F 9/24**, A61K 31/66, C07H 13/04, A61K 31/70

(30) Priorität: **31.01.92 DE 4202706**

(43) Veröffentlichungstag der Anmeldung: **04.08.93 Patentblatt 93/31**

(84) Benannte Vertragsstaaten: **AT BE CH DE DK ES FR GB GR IE IT LI LU MC NL PT SE**

(71) Anmelder: **BAYER AG**
**W-5090 Leverkusen 1 Bayerwerk(DE)**

(72) Erfinder: **Tietze, Lutz F., Dr. Prof.**
**Stumpfe Eiche 73**
**W-3400 Göttingen(DE)**
Erfinder: **Graf, Susanne**
**Ginsterweg 18**
**W-3400 Göttingen(DE)**

## (54) N,N-Bis-(2-chlorethyl) phosphorsäurediamidate als Cytostatika.

(57) Die Erfindung betrifft neue N,N-Bis-(2-Chlorethyl)phosphorsäurediamidate, Verfahren zu ihrer Herstellung und ihre Verwendung als hochselektive Cytostatika in der Krebstherapie.

EP 0 553 673 A1

Die Erfindung betrifft neue N,N-Bis-(2-Chlorethyl)phosphorsäurediamidate, Verfahren zu ihrer Herstellung und ihre Verwendung als hochselektive Cytostatika in der Krebstherapie.

Es ist bekannt, daß maligne Zellen eine gegenüber dem Normalgewebe erhöhte Glycolyse und damit Lactatproduktion aufzeigen und der pH-Wert im Tumorgewebe durch intravenös verabreichte Glucose gesenkt werden kann (vgl. S. Tanneberger, Experimentelle und klinische Tumorchemotherapie; Allgemeine Tumorchemotherapie; G. Fischer Verlag, Stuttgart/New York 1980; Naturwiss. 46, 2(1959); Cancer Res. 42, 1498 (1982); 42, 1505 (1982)).

In der Vergangenheit wurde versucht, diese Unterschiede im pH-Wert zwischen normalem und Tumorgewebe für eine selektive Tumortherapie auszunutzen (vergl. Liebigs Ann. Chem. 1987, 847-856; Tetrahedron Lett. 22 (1981) 239- 242). Es war angestrebt worden, alkylierende Verbindungen, die wegen einer zu geringen Differenzierung zwischen gesundem und malignem Gewebe eine sehr geringe therapeutische Breite aufweisen, in untoxische, säurelabile Prodrug-Formen zu überführen, die nur im Tumorgewebe aufgrund des dort vorherrschenden erniedrigten pH-Wertes selektiv gespalten werden zum aktiven, alkylierend wirkenden Cytostatikum. Auf diese Weise sollte eine selektive Tumortherapie angestrebt werden.

Es hatte sich aber gezeigt, daß die in der oben angeführten Literaturstelle hergestellten Verbindungen sich nicht als so säurelabil erwiesen, als daß sie selektiv im Tumorgewebe zum aktiven cytociden Agenz rückgespalten wurden.

Es wurde nun überraschenderweise festgestellt, daß die folgenden genannten Verbindungen untoxisch sind, während sie bei dem im Tumorgewebe durch Hyperglykämie erreichbaren pH-Wert durch Hydrolyse in cytocide Verbindungen überführt werden können.

Die vorliegende Erfindung betrifft N,N-Bis-(2-Chlorethyl)phosphorsäurediamidate der allgemeinen Formel (I)

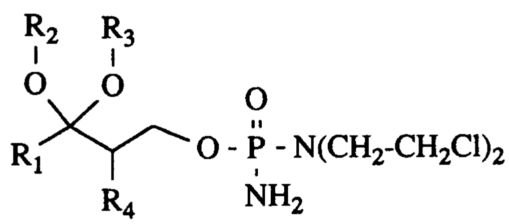 (I)

in welcher

$R^1$ für einen Zuckerrest der Formel

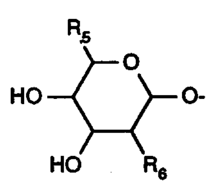 oder 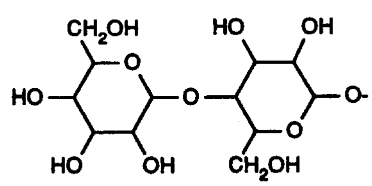

in der D- oder L-Form steht,
worin

$R^5$ Methyl oder die $-CH_2OH$-Gruppe bedeutet
und

$R^6$ die $-CH_2OH$-Gruppe oder einen Rest der Formel $-NR^7R^8$ bedeutet,
worin

$R^7$ und $R^8$ gleich oder verschieden sind und Wasserstoff oder eine Aminoschutzgruppe bedeuten,
wobei die Hydroxygruppen der Zuckerreste gegebenenfalls geschützt sind,
oder
für geradkettiges oder verzweigtes Alkyl mit bis zu 8 Kohlenstoffatomen steht, das gegebenenfalls bis zu 2-fach gleich oder verschieden durch Hydroxy, geschütztes Hydroxy oder geradkettiges oder verzweigtes Alkoxy mit bis zu 6 Kohlenstoffatomen,

Benzyl oder Benzyloxy oder einen der oben aufgeführten Zuckerreste der Formeln

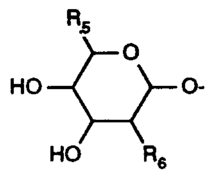

oder

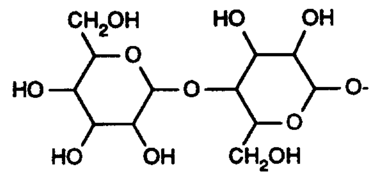

in der D- oder L-Form substituiert ist,
worin

$R^5$ und $R^6$ die oben angegebene Bedeutung haben,

$R^2$ und $R^3$ gleich oder verschieden sind und für Aryl mit 6 bis 10 Kohlenstoffatomen stehen für geradkettiges oder verzweigtes Alkyl mit bis zu 8 Kohlenstoffatomen stehen, das gegebenenfalls durch Halogen, Hydroxy, geschütztes Hydroxy, geradkettiges oder verzweigtes Alkoxy mit bis zu 8 Kohlenstoffatomen oder durch eine Gruppe der Formel $-NR^9R^{10}$ substituiert ist,
worin

$R^9$ und $R^{10}$ gleich oder verschieden sind und Wasserstoff, geradkettiges oder verzweigtes Alkyl mit bis zu 6 Kohlenstoffatomen oder eine Gruppe der Formel $-CO-R^{11}$ bedeuten,
worin

$R^{11}$ geradkettiges oder verzweigtes Alkyl mit bis zu 6 Kohlenstoffatomen oder Trifluormethyl bedeutet,

$R^4$ für Wasserstoff oder für geradkettiges oder verzweigtes Alkyl mit bis zu 8 Kohlenstoffatomen steht,

oder

$R^1$ und $R^4$ gemeinsam einen 5- bis 7-gliedrigen, gesättigten Carbocyclus bilden.

Hydroxyschutzgruppe im Rahmen der oben angegebenen Definition steht im allgemeinen für eine Schutzgruppe aus der Reihe: tert.Butoxydiphenylsilyl, Trimethylsilyl, Triethylsilyl, Triisopropylsilyl, tert.Butyldimethylsilyl, tert.Butyldiphenylsilyl, Triphenylsilyl, Trimethylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert. Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methylthiomethyl, Methoxyethoxymethyl, [2-(Trimethylsilyl)ethoxy]methyl, 2-(Methylthiomethoxy)ethoxycarbonyl, Benzoyl, 4-Methylbenzoyl, 4-Nitrobenzoyl, 4-Fluorbenzoyl, 4-Chlor-4-Chlorbenzoyl oder 4-Methoxybenzoyl. Bevorzugt sind Acetyl, Benzoyl, Benzyl oder Methylbenzyl.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tert.butoxycarbonyl, Menthyloxycarbonyl, Phenoxycarbonyl, 4-Nitrophenoxycarbonyl, 9-Fluorenyl-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethyl, 4-Nitrobenzyl, 2,4-Dinitrobenzyl oder 4-Nitrophenyl. Bevorzugt sind Benzyloxycarbonyl und Acetyl.

Bevorzugt sind Verbindungen der allgemeinen Formel (I),
in welcher

$R^1$ für einen Zuckerrest der Formel

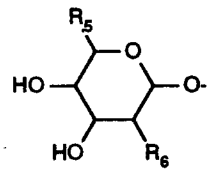 oder 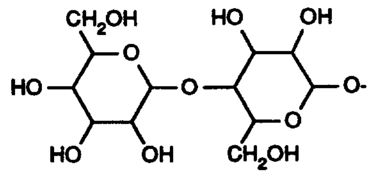

in der D- oder L-Form steht, worin

$R^5$ Methyl oder die $-CH_2OH$-Gruppe bedeutet

und

$R^6$ die OH-Gruppe oder einen Rest der Formel $-NR^7R^8$ bedeutet,

worin

$R^7$ und $R^8$ gleich oder verschieden sind und Wasserstoff oder Acetyl bedeuten,

wobei die Hydroxygruppen der Zuckerreste gegebenenfalls durch Acetyl, Benzyl oder Methylbenzoyl geschützt sind,

für geradkettiges oder verzweigtes Alkyl mit bis zu 6 Kohlenstoffatomen steht, das gegebenenfalls durch Hydroxy oder durch geschütztes Hydroxy, geradkettiges oder verzweigtes Alkoxy mit bis zu 4 Kohlenstoffatomen, Benzyl oder durch einen der oben aufgeführten Zuckerreste der Formel

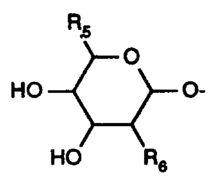 oder 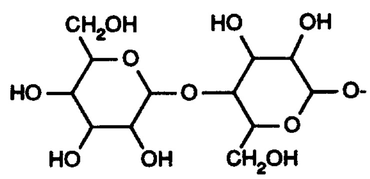

in der D- oder L-Form substituiert ist,

worin

$R^5$ und $R^6$ die oben angegebene Bedeutung haben,

$R^2$ und $R^3$ gleich oder verschieden sind und für Phenyl oder geradkettiges oder verzweigtes Alkyl mit bis zu 6 Kohlenstoffatomen stehen, das gegebenenfalls durch Fluor, Chlor, Brom, Hydroxy, geradkettiges oder verzweigtes Alkoxy mit bis zu 6 Kohlenstoffatomen oder durch eine Gruppe der Formel $-NR^9R^{10}$ substituiert ist,

worin

$R^9$ und $R^{10}$ gleich oder verschieden sind und Wasserstoff, geradkettiges oder verzweigtes Alkyl mit bis zu 4 Kohlenstoffatomen oder eine Gruppe der Formel $-CO-R^{11}$ bedeuten,

worin

$R^{11}$ geradkettiges oder verzweigtes Alkyl mit bis zu 4 Kohlenstoffatomen oder Trifluormethyl bedeutet,

$R^4$ für Wasserstoff oder für geradkettiges oder verzweigtes Alkyl mit bis zu 6 Kohlenstoffatomen steht,

oder

$R^1$ und $R^4$ gemeinsam einen Cyclopentyl- oder Cyclohexylring bilden.

Besonders bevorzugt sind Verbindungen der allgemeinen Formel (I),

in welcher

$R^1$ für einen Zuckerrest der Formel

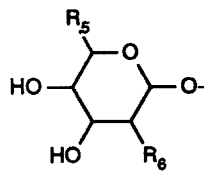 oder 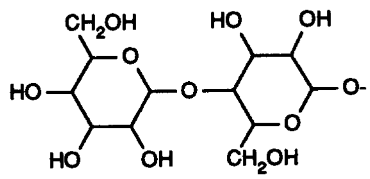

in der D- oder L-Form steht,
worin

$R^5$ die $-CH_2OH$-Gruppe bedeutet
und

$R^6$ die OH-Gruppe oder einen Rest der Formel $-NR^7R^8$ bedeutet,
worin

$R^7$ und $R^8$ gleich oder verschieden sind und Wasserstoff oder Acetyl bedeuten,
wobei die Hydroxygruppen der Zuckerreste gegebenenfalls durch Acetyl, Benzyl oder Methylbenzoyl geschützt sind,
für geradkettiges oder verzweigtes Alkyl mit bis zu 4 Kohlenstoffatomen steht, das gegebenenfalls durch Hydroxy, Methoxy, Ethoxy, Propoxy, Isopropoxy, Benzyl, Benzyloxy oder durch einen der oben aufgeführten Zuckerreste der Formel

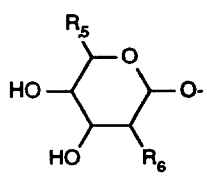 oder 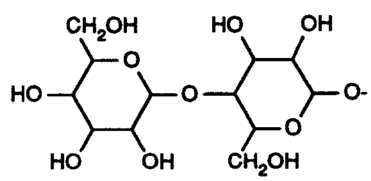

in der D- oder L-Form substituiert ist,
worin

$R^5$ und $R^6$ die oben angegebene Bedeutung haben,

$R^2$ und $R^3$ gleich oder verschieden sind und für Phenyl oder geradkettiges oder verzweigtes Alkyl mit bis zu 4 Kohlenstoffatomen stehen, das gegebenenfalls durch Fluor, Chlor, Brom, Hydroxy, geradkettiges oder verzweigtes Alkoxy mit bis zu 4 Kohlenstoffatomen oder durch eine Gruppe der Formel $-NR^9R^{10}$ substituiert ist,
worin

$R^9$ und $R^{10}$ gleich oder verschieden sind und Wasserstoff, Methoxy, Ethyl, Propyl, Isopropyl oder eine Gruppe der Formel $-CO-R^{11}$ bedeuten,
worin

$R^{11}$ Methyl, Ethyl oder Trifluormethyl bedeutet

$R^4$ für Wasserstoff oder für geradkettiges oder verzweigtes Alkyl mit bis zu 4 Kohlenstoffatomen steht,

oder

$R^1$ und $R^4$ gemeinsam einen Cyclopentyl- oder Cyclohexylring bilden.

Außerdem wurde ein Verfahren zur Herstellung der erfindungsgemäßen Verbindungen der allgemeinen Formel (I) gefunden, dadurch gekennzeichnet, daß man

Verbindungen der allgemeinen Formel (II)

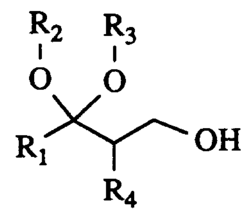

(II)

in welcher

$R^1$, $R^2$, $R^3$ und $R^4$ die oben angegebene Bedeutung haben,

zunächst mit N,N-Bis-(2-Chlorethyl)phosphorsäureamiddichlorid der Formel (III)

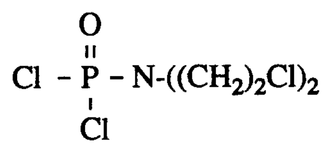

(III)

in inerten Lösemitteln, in Anwesenheit einer Base und gegebenenfalls eines Hilfsstoffes, umsetzt, anschließend eine Ammonolyse durchführt,

und im Fall, daß $R^1$ und/oder einer der oben aufgeführten Zuckerreste eine Schutzgruppe trägt, diese nach üblicher Methode abspaltet.

Das erfindungsgemäße Verfahren kann durch folgendes Formelschema beispielhaft erläutert werden:

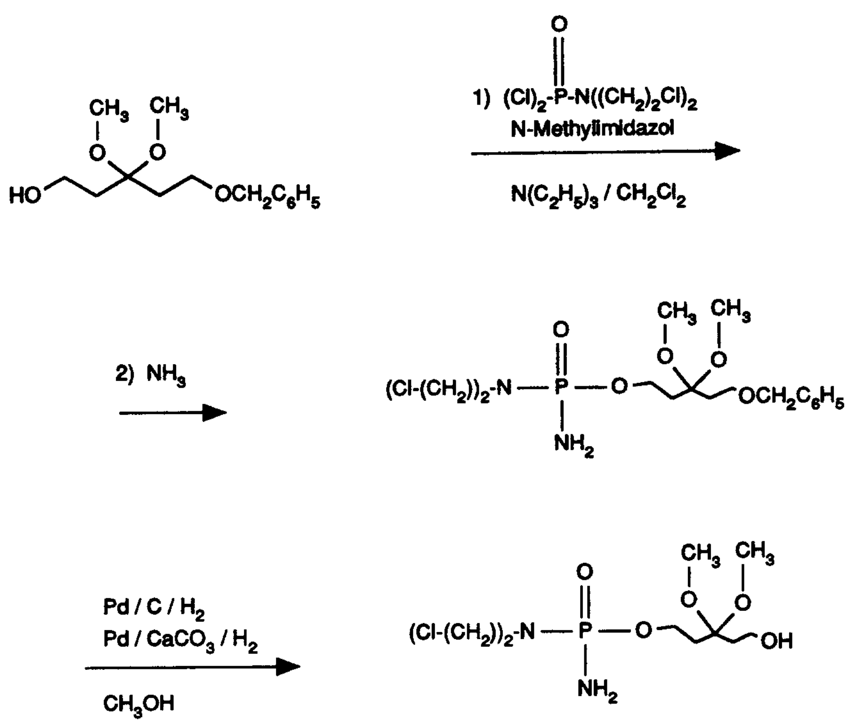


Als Lösemittel eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan, Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid, Acetonitril, Aceton oder Nitromethan. Ebenso ist es möglich, Gemische der genannten Lösemittel zu verwenden. Bevorzugt sind Tetrahydrofuran, Dichlormethan und Toluol.

Als Basen eignen sich 1,8-Diazabicyclo[5.4.0]undec-7-en, Imidazol, Pyridin, Tetrazol und die üblichen organischen Amine. Hierzu gehören bevorzugt Alkylamine wie Triethylamin, Diisopropylamin, Dicyclohexylamin und Ethyldiisopropylamin. Besonders bevorzugt sind Triethylamin, 1,8-Diazabicyclo[5.4.0]undec-7-en und Pyridin.

Als Hilfstoffe eignen sich N-Methylimidazol und Dimethylaminopyridin (DMAP).

Die Basen und Hilfsstoffe werden im allgemeinen in einer Menge von 0,01 bis 5 mol, bevorzugt von 0,03 bis 1,75 mol, jeweils bezogen auf 1 Mol der Verbindungen der allgemeinen Formel (III) eingesetzt.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Das erfindungsgemäße Verfahren wird im allgemeinen in einem Temperaturbereich von 0 °C bis +60 °C, bevorzugt bei Raumtemperatur durchgeführt.

Die oben aufgeführten Reaktionsbedingungen werden auch während der Ammonolyse nicht verändert. Das durch die Durchleitung des Ammoniakstroms verdampfte Lösemittel wird während der Umsetzung ersetzt.

Die Abspaltung der Schutzgruppen am Zuckerrest erfolgt nach üblicher Methode in inerten Lösemitteln in Anwesenheit einer Base oder durch Hydrogenolyse.

Als Basen eignen sich für die Abspaltung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumcarbonat oder Kaliumcarbonat eingesetzt, aber auch basische Ionenaustauscher, wie z.B. Lewatit®UL500.

Als Lösemittel eignen sich für die Abspaltung die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Abspaltung wird im allgemeinen in einem Temperaturbereich von 0 °C bis +100 °C, bevorzugt von +20 °C bis +40 °C durchgeführt. Im allgemeinen wird die Abspaltung bei Normaldruck durchgeführt. Es ist aber auch möglich, bei Unterdruck oder bei Überdruck zu arbeiten (z.B. von 0,5 bis 5 bar).

Die Abspaltung von speziellen Hydroxyschutzgruppen (Silyl- und Benzylgruppen) erfolgt beispielsweise mit Tetrabutylammoniumfluorid oder durch hydrogenolytische Spaltung, im Fall der Benzylgruppe in Anwesenheit eines Katalysators, beispielsweise ein Gemisch aus Palladium/C und Palladium/$CaCO_3$, mit Wasserstoff, in einem der oben aufgeführten Lösemittel, vorzugsweise Methanol, in einem Temperaturbereich von 0 °C bis 40 °C, vorzugsweise bei Raumtemperatur.

Die Abspaltung der Aminoschutzgruppen wird im allgemeinen ebenfalls nach bekannten Methoden, beispielsweise mit Kaliumcarbonat in Methanol durchgeführt [vgl. außerdem Th. Greene: "Protective Groups in Organic Synthesis", J. Wiley & Sons, 1981, New York].

N,N-Bis-(2-chlorethyl)-phosphorsäureamiddichlorid der Formel (III) ist bekannt [vgl. J. Am. Chem. Soc. 76, 655 (1954)].

Die Verbindungen der allgemeinen Formel (II) sind teilweise bekannt oder neu und können dann hergestellt werden, indem man entweder

[A] Ketone der allgemeinen Formel (IV)

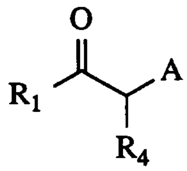

(IV)

in welcher

$R^1$ und $R^4$ die oben angegebene Bedeutung haben
und
A entweder für $C_1$-$C_4$-Alkoxycarbonyl oder für einen Rest der Formel $-CH_2-OD$ steht, worin
D eine der oben aufgeführten Silylschutzgruppen bedeutet,
zunächst durch Umsetzung mit Verbindungen der allgemeinen Formel (V)

$R^{12}$-O-E (V)

in welcher
E die oben angegebene Bedeutung von D hat und mit dieser gleich oder verschieden ist,
$R^{12}$ die oben angegebene Bedeutung von $R^2$ und $R^3$ hat,
unter Abspaltung der Schutzgruppe E, in inerten Lösemitteln acetalisiert und anschließend entweder eine Reduktion (A = $C_1$-$C_4$-Alkoxycarbonyl) oder eine Abspaltung des Silylrestes (D) nach üblichen Methoden durchführt,
oder

[B] Enolether der allgemeinen Formel (VI)

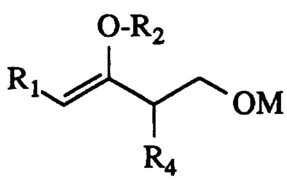

(VI)

in welcher

$R^1$, $R^2$ und $R^4$ die oben angegebene Bedeutung haben
und
M für eine der oben aufgeführten Silylschutzgruppen, vorzugsweise für tert.Butyldiphenylsilyl steht,
zunächst mit Verbindungen der allgemeinen Formel (VII)

$R^3$-OH (VII)

in welcher
$R^3$ die oben angegebene Bedeutung hat,
acetalisiert und anschließend nach der oben aufgeführten Methode desilyliert,
oder Enoletherderivate der allgemeinen Formel (VIa)

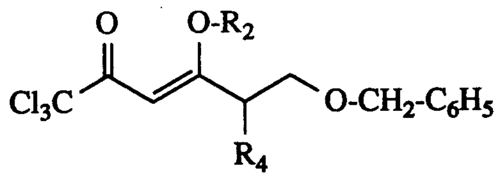

(VIa)

in welcher

$R^2$ und $R^4$ die oben angegebene Bedeutung haben,

durch Umsetzung mit Verbindungen der allgemeinen Formel (VII) unter Acetalisierung und Haloformierung in die Verbindungen der allgemeinen Formel (VIII)

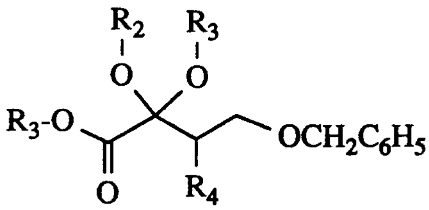

(VIII)

in welcher

$R^2$, $R^3$ und $R^4$ die oben angegebene Bedeutung haben

überführt und in einem letzten Schritt die Alkoxycarbonylfunktion ($-CO_2CH_3$) reduziert.

Für die Verfahren [A] und [B] eignen sich die oben aufgeführten Lösemittel und Basen. Bevorzugt sind als Lösemittel Methylenchlorid und als Basen Pyridin und Triethylamin.

Die Reduktion der Alkoxycarbonylfunktionen erfolgt im allgemeinen mit Hydriden, bevorzugt mit Lithiumaluminiumhydrid in inerten Lösemitteln wie Ethern, Kohlenwasserstoffen oder Halogenkohlenwasserstoffen oder deren Gemischen, bevorzugt in Ethern wie beispielsweise Diethylether, Tetrahydrofuran oder Dioxan in einem Temperaturbereich von 0 °C bis +150 °C, bevorzugt von +20 °C bis +60 °C, bei Normaldruck. Bevorzugt ist die Reduktion mit Lithiumaluminiumhydrid in Ether.

Die Desilylierung erfolgt neben der oben aufgeführten Methode bevorzugt mit Tetra-n-butylammoniumfluorid/$H_2O$.

Die Verbindungen der allgemeinen Formeln (IV), (V), (VI) und (VII) sind an sich bekannt oder können nach üblicher Methode hergestellt werden.

Die Verbindungen der allgemeinen Formel (VIa) sind neu und können aus den entsprechenden Enolethern durch Umsetzung mit Trichloracetylchlorid in Pyridin hergestellt werden.

In Abhängigkeit von den Reaktionsbedingungen der einzelnen Verfahrensschritte ist es notwendig, diese unter Schutzgasatmosphäre durchzuführen.

Die erfindungsgemäßen Verbindungen zeigen ein nicht vorhersehbares, wertvolles pharmakologisches Wirkspektrum.

Die säurelabilen Derivate des inaktivierten Cyclophosphamids der allgemeinen Formel (I) stellen erstmalig ein Cytostatikum dar, das in seiner Transportform untoxisch ist. Bei dem im Tumorgewebe durch Hyperglykämie erreichbaren pH-Wert setzen sie durch Hydrolyse eine cytocide Verbindung, die Friedman-Säure, frei.

2,7 mg der zu spaltenden Verbindung wurden in 240 µl [$D_8$]-Dioxan, 210 µl $D_2O$ sowie 150 µl Pufferlösung (Oxalsäure / Natriumoxalat-Puffer des pD-Wertes 4,78) gelöst. Dabei wurde auf 35 °C thermostatiert. Es wurden bei pD 4,78 insgesamt 12 Spektren registriert, wobei die ersten vier Spektren in Zeitintervallen von je 12 min gemessen wurden. Danach betrugen die Zeitintervalle ca. 20 min. Die Konzentration des verwendeten Oxalatpuffers betrug 0,025 M.

Überprüfung der cytotoxischen Wirksamkeit im Zellkulturtest

Mit den Ketophosphamid-Prodrugs (RS-P)-1-0-(3,3-Dimethoxy)butyl-N,N-bis-(2-chlorethyl)-phosphorsäurediamidat (S. 34, Tabelle 1, Beispiel 5) und (RS-P)-1-0-(5-Benzyloxy-3,3-dimethoxy)pentyl-N,N-bis(2-chlorethyl)phosphorsäurediamidat (S. 34, Beispiel 6) wurden Untersuchungen an Krebszellen der Zellinie BIR-M1$R_k$, die dem Mamma-Karzinom von Marshall-Ratten entstammen, durchgeführt und die Überlebensrate der Zellen in Abhängigkeit vom pH-Wert des Mediums und der Dosis untersucht. Bei pH 7,4 als Modell für eine Normalzellpopulation wurde nur eine geringe Toxizität, bei pH 6,2 als Modell für eine Krebszellpopulation unter den Bedingungen der Hyperglykämie hingegen eine um den Faktor 10 000 verringerte Überlebensrate der Krebszellen bei einer Dosis von 10 µg/ml gefunden.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 150 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 100, insbesondere 1 bis 80 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Ausgangsverbindungen

Beispiel I

4-tert.Butyldiphenylsilyloxybutan-2-on

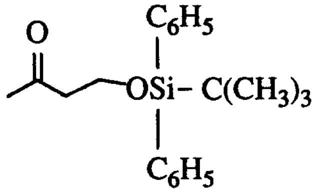

Als Ausgangsverbindung dient das 4-Hydroxybutan-2-on, das in großem Maßstab durch Kondensation von Aceton mit Paraformaldehyd erhältlich ist [vgl. J. Am. Chem. Soc. 73, (1951), 5369]. Die nachfolgende Silylierung erfolgt entsprechend der Vorschrift von P. Lavalle und S. Hanessian, Can. J. Chem. 53, (1975), 2975, in leicht modifizierter Weise (Beller, Dissertation, Universität Göttingen 1989).

Beispiel II

4-(tert.Butyldiphenylsiloxy)-2-trimethylsiloxybut-1-en

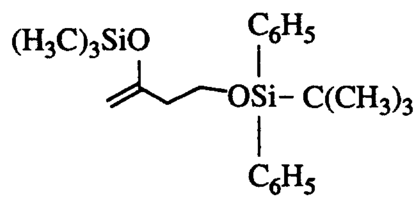

3,49 ml (24,9 mmol) Diisopropylamin werden in 100 ml abs. Tetrahydrofuran gelöst und auf -78 °C abgekühlt. Innerhalb von 10 min werden 13,5 ml (21,6 mmol) einer 1,6 ml n-Butyllithium-Lösung in n-Hexan zugetropft. Man läßt auf 0 °C kommen, rührt 30 min bei dieser Temperatur und kühlt erneut auf -78 °C ab, um eine Lösung aus 6,67 g (20,4 mmol) der Verbindung aus Beispiel I und 5 ml Tetrahydrofuran langsam zuzugeben. Nach einstündigem Rühren bei -78 °C wird vorsichtig mit 4,93 ml eines Gemisches aus Trimethylsilylchlorid und Triethylamin versetzt (Herstellung des Silylierungsreagenzes: Equimolare Mengen Trimethylsilylchlorid und Triethylamin wurden 1 h zentrifugiert Die überstehende Lösung wird als Silylierungsmittel eingesetzt [vgl. hierzu J. Am. Chem. Soc. 96, (1974), 6181]). Man läßt die Reaktionslösung auf -15 °C kommen, rührt 15 min und anschließend nochmals 30 min bei Raumtemperatur. Nach beendeter Umsetzung wird das Reaktionsgemisch mit 100 ml n-Pentan versetzt und dreimal mit je 150 ml eiskalter gesättigter Natriumhydrogencarbonatlösung gewaschen. Man trocknet die organische Phase über Natriumsulfat, filtriert und entfernt das Lösemittel im Vakuum. Die Vakuumdestillation liefert 5,70 g (70%) des reinen Silylenolethers als farblose Flüssigkeit.
Sdp.: 132 °C/0,01 Torr
$R_f$ = 0,89 (tert.Butylmethylether / Petrolether 1:10)

Beispiel III

(5 RS)-5-Ethoxy-1-(tert.butyldiphenylsiloxy)hexan-3-on

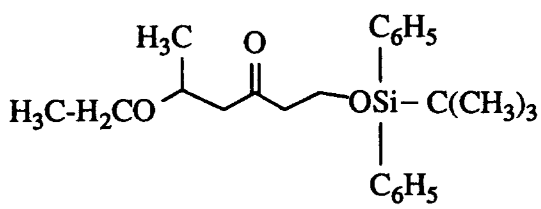

398 mg (1,00 mmol) der Verbindung aus Beispiel II und 130 mg (1,10 mmol) Acetaldehyddiethylacetal werden in 3 ml abs. Dichlormethan gelöst und auf -78 °C abgekühlt. Man spritzt 9,1 µl TMS-Triflat als Katalysator zu und rührt 10 h bei dieser Temperatur. Anschließend wird die Reaktion bei -78 °C durch Zugabe von 2 ml Methanol / Triethylamin 1:1 abgebrochen. Man verdünnt mit 20 ml Dichlormethan und extrahiert das Reaktionsgemisch zweimal mit je 30 ml eiskalter gesättigter Natriumhydrogencarbonatlösung. Anschließend trocknet man die organische Phase durch Filtration über Kaliumcarbonat, wäscht mit 20 ml Dichlormethan nach, entfernt das Lösemittel im Vakuum und reinigt den Rückstand durch Flashchromatographie an 50 g Kieselgel (32 - 64 µm) in tert.Butylmethylether/Petrolether 1:10. Man erhält 643 mg (52%) des gewünschten Ketons als farbloses Öl.
$R_f$ = 0,22 (Essigsäureethylester / Petrolether 1:10)

Beispiel IV

2-Methoxy-4-(tert.butoxydiphenylsiloxy)but-1-en

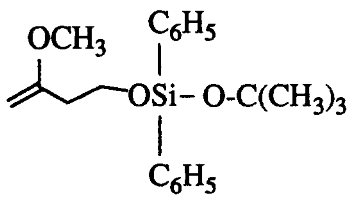

Ausgehend von Methylvinylether, der in Hexan/Tetrahydrofuran bei -80 °C mittels Schlosser-Reagenz [a] in α-Stellung deprotoniert wird, erhält man durch Umsetzung mit Ethylenoxid bei -40 °C 4-Hydroxy-2-methoxybut-1-en [b]. Nachfolgende Silylierung mit der tert.Butoxydiphenylsilyl-Schutzgruppe in Gegenwart von Triethylamin liefert den geschützten Enolether in 79%iger Ausbeute [vgl. hierzu a) Preparative Polar Organometallic Chemistry, Springer, Berlin 1987, S. 76 ff., J. Am. Chem. Soc. 98 (1976), 4674; b) J.W. Gillard et al., J. Org. Chem. 53, (1988), 2603].

Beispiel V

3,3-Dimethoxybutansäureethylester

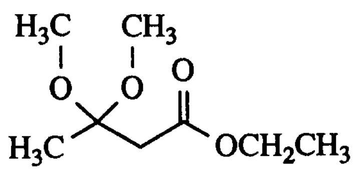

140 g K 10-Montmorillonit-Clay werden mit 204 g (1,93 mol, 211 ml) Trimethylorthoformiat 10 min bei 20 °C kräftig gerührt. Man filtriert und gibt den nassen Filterkuchen in einen 1 l-Dreihalskolben. Dazu wird eine Lösung von 60,9 g (0,47 mol) Acetessigsäureethylester in 420 ml Petrolether gegeben und 5 min (maximal) bei Raumtemperatur schnell gerührt. Anschließend wird der Katalysator abgesaugt (es darf keine Spur des Katalysators in der Lösung bleiben) und der Filterkuchen mit Petrolether nachgewaschen. Die vereinigten Filtrate werden im Vakuum auf die Hälfte eingeengt, mit gesättigter wäßriger Natriumhydrogencarbonatlösung gewaschen und über Natriumsulfat getrocknet. Nach Filtration und Abdampfen des Lösemittels destilliert man den Rückstand im Vakuum über eine 20 cm-Vigreuxkolonne. Man erhält 58,4 g (71%) der Titelverbindung als klare, farblose Flüssigkeit vom Sdp. 75 °C / 10 Torr.

Beispiel VI

(1 RS)-2,2-Dimethoxycyclohexan-1-carbonsäureethylester

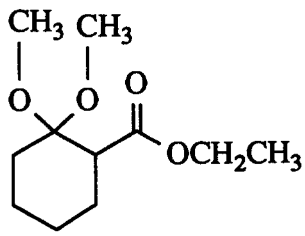

2,00 g (12,0 mmol) Cyclohexanon-2-carbonsäureethylester werden unter Feuchtigkeitsausschluß in 5 ml abs. Methanol gelöst und mit 1,27 g (12,0 mmol) Trimethylorthoformiat versetzt. Anschließend gibt man 1,2 ml einer methanolischen Salzsäurelösung zu, erhitzt 2 h unter Rückfluß und rührt noch 15 h bei Raumtemperatur. Nach beendeter Umsetzung (DC-Kontrolle) wird das Reaktionsgemisch mit 50 ml Ether verdünnt und dreimal mit je ca. 40 ml eiskalter gesättigter Natriumhydrogencarbonatlösung gewaschen. Trocknen der organischen Phase über Natriumsulfat, Filtration und Abdampfen des Lösemittels im Vakuum liefern das Rohprodukt, welches durch Flashchromatographie an 130 g Kieselgel (32 - 64 µm) in Essigsäureethylester / Petrolether 1:10 mit 0,7% Triethylamin gereinigt wird. Man erhält 2,51 g (73%) des gewünschten Acetals.
$R_f$ = 0,34 (Essigsäureethylester / Petrolether 1:8)

Beispiel VII

(5 RS)-3,3-Dimethoxy-5-ethoxy-1-(tert.butyldiphenylsiloxy)hexan

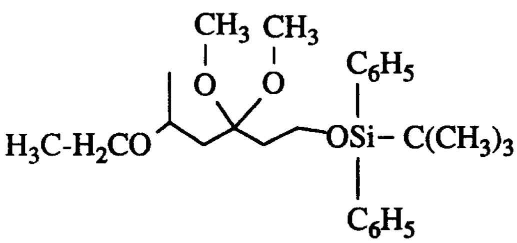

Unter Feuchtigkeitsausschluß und unter Schutzgas werden 701 mg (1,76 mmol) der Verbindung aus Beispiel III und 440 mg (4,22 mmol) Methyltrimethylsilylether in 4 ml abs. Dichlormethan gelöst und auf -78°C abgekühlt. Man spritzt 96,1 µl (0,53 mmol) TMS-Triflat zu und läßt 24 h bei dieser Temperatur reagieren. Der Abbruch der Reaktion erfolgt durch Zugabe von 2 ml Triethylamin / Methanol 1:1. Abdampfen des Lösemittels im Vakuum sowie Flashchromatographie an 75 g Kieselgel (32 - 64 µm) in Essigsäureethylester / Petrolether 1:12 mit 0,7% Triethylamin liefern 312 mg (40%) der Titelverbindung.
$R_f$ = 0,28 (tert.Butylmethylether / Petrolether 1:10)

Beispiel VIII

2,2-Bis-(2-methoxyethoxy)-4-(tert.butyldiphenylsiloxy)butan

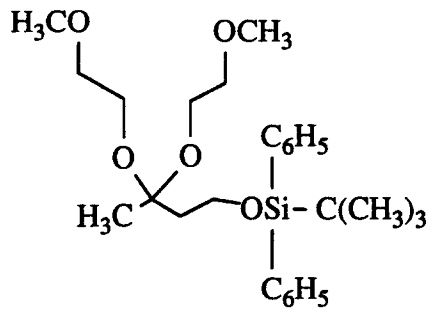

Analog der Vorschrift für Beispiel VII erfolgt die Darstellung der Titelverbindung unter Verwendung von 2-Methoxyethyltrimethylsilylether.

Beispiel IX

2-Methoxy-2-(2-methoxyethoxy)-4-(tert.butoxydiphenylsiloxy)butan

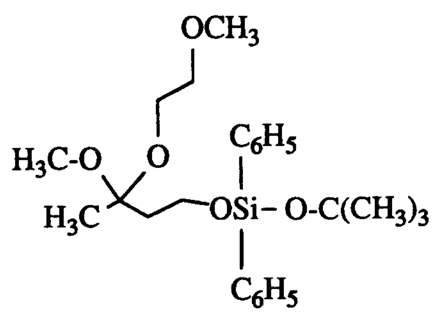

Unter Feuchtigkeitsausschluß und unter Schutzgas werden 100 mg pulverisiertes Molsieb 4 A sowie 126 mg (0,50 mmol) Pyridinium-p-toluolsulfonat in 3 ml abs. Dichlormethan vorgelegt und mit 927 mg (2,6 mmol) 2-Methoxy-4-(tert.butoxydiphenylsiloxy)but-1-en versetzt. Man kühlt auf 0 °C ab und tropft eine Lösung aus 152 mg (2,0 mmol) Ethylenglycolmonomethylether in 1 ml abs. Dichlormethan langsam zu. Nach 45 min zeigt DC-Kontrolle vollständigen Umsatz an. Man filtriert die Reaktionsmischung über ca. 10 g Alox B super-Stufe 1 und wäscht mit 150 ml tert.Butylmethylether nach. Abdampfen des Lösemittels im Vakuum liefert das Rohprodukt, welches anschließend durch Flashchromatographie an 75 g Kieselgel (32 - 64 µm) in tert.Butylmethylether / Petrolether 1:12 mit 0,7% Triethylamin gereinigt wird. Man erhält 346 mg (40%) einer farblosen Flüssigkeit.
$R_f$ = 0,21 (tert.Butylmethylether / Petrolether 1:10)

Beispiel X

2-(2-Bromethyl)-2-methoxy-4-(tert.butoxydiphenylsiloxy)butan

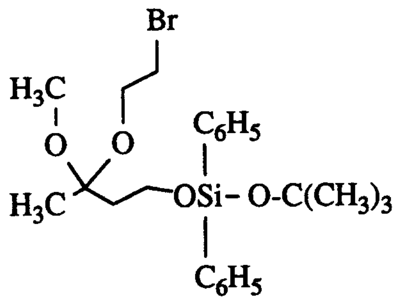

Analog der Vorschrift für Beispiel IX erfolgt die Darstellung der Titelverbindung unter Verwendung von 2-Bromethanol.

Beispiel XI

4-Benzyloxy-2-methoxybut-1-en

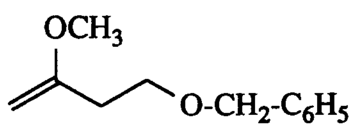

Unter Feuchtigkeitsausschluß und Schutzgas werden 2,00 g (81,0 mmol) Natriumhydrid in 140 ml wasserfreiem Tetrahydrofuran als Lösemittel vorgelegt. Unter Rühren und unter Eiskühlung tropft man 5,50 g (54,0 mmol) 4-Hydroxy-2-methoxybut-1-en hinzu und rührt 15 min bei 0 °C. Anschließend wird das Reaktionsgemisch nacheinander mit 283 mg Tetra-n-butylammoniumjodid sowie 6,25 ml (52,0 mmol) Benzylbromid versetzt. Man rührt erneut 15 min bei 0 °C, danach 2 h bei Raumtemperatur (DC-Kontrolle). Nach Zugabe von 10 g Alox B super-Stufe 1 zum Reaktionsgemisch wird die überstehende Lösung vom Niederschlag abdekantiert und dieser zweimal mit je 80 ml Ether gewaschen. Man filtriert die vereinigten Extrakte über ca. 20 g Alox B super-Stufe 1 (Ether), trocknet über Natriumsulfat und trennt vom Trockenmittel ab. Abdampfen des Lösemittels im Vakuum lieferte 8,92 g (86%) der Titelverbindung als leicht gelb gefärbtes Öl, welches direkt in die weitere Synthese eingesetzt wird.
$R_f$ = 0,45 (Essigsäureethylester / Petrolether 1:8)

Beispiel XII

1,1,1-Trichlor-6-benzyloxy-4-methoxyhexen-2-on

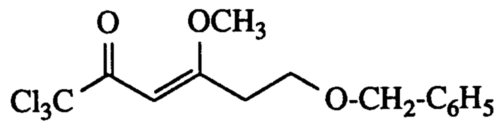

Unter Feuchtigkeitsausschluß und Schutzgas werden 2,94 g (16,2 mmol) Trichloracetylchlorid mit 5 ml Pyridin versetzt und mittels Eis-Kochsalz-Bad auf -5 °C gebracht. Anschließend tropft man 4,00 g (21,0 mmol) der Verbindung aus Beispiel XI zu und entfernt das Kühlbad nach beendeter Zugabe. Nach 15 stündiger Reaktionszeit bei Raumtemperatur (DC-Kontrolle) werden 40 ml Wasser zugegeben. Man extrahiert das Gemisch zweimal mit je 50 ml Ether, trocknet die organische Phase über Natriumsulfat, filtriert und dampft das Lösemittel im Vakuum ab. Man erhält 5,92 g eines gelben Öls als Rohprodukt, welches direkt in die weitere Synthese eingesetzt wird.
$R_f$ = 0,43 (Essigsäureethylester / Petrolether 1:4)

Beispiel XIII

5-Benzyloxy-3,3-dimethoxypentansäuremethylester

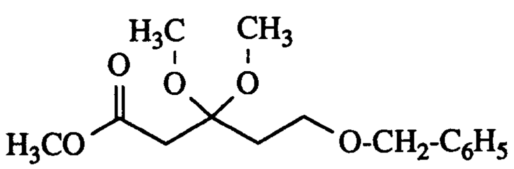

5,92 g der Verbindung aus Beispiel XII (Rohprodukt der Effenberger-Reaktion) werden in 6 ml wasserfreiem Methanol gelöst und unter Schutzgas bei Raumtemperatur zu einer Mischung aus 10 Tropfen 1,8-Diazabicyclo[5.4.0]undec-7-en und 2 ml wasserfreiem Methanol zugetropft. Nach 15 stündigem Rühren bei Raumtemperatur zeigt dünnschichtchromatographische Kontrolle, daß sich das Edukt nicht vollständig umgesetzt hat. Man versetzt deshalb nochmals mit 6 Tropfen 1,8-Diazabicyclo[5.4.0]undec-7-en, rührt weitere 5 h bei Raumtemperatur und arbeitet anschließend wie folgt auf: Das Reaktionsgemisch wird mit 50 ml Ether verdünnt und zweimal mit je 80 ml einer eiskalten gesättigten Natriumhydrogencarbonatlösung gewaschen. Man trocknet über Natriumsulfat und filtriert vom Trockenmittel ab. Abdampfen des Lösemittels im Vakuum liefert 4,35 g eines braunen Öls als Rohprodukt, das ohne weitere Reinigung in die nachfolgende Reaktion eingesetzt wird.
Anmerkung: bei größeren Ansätzen reagiert das Edukt auch nach weiterer 1,8-Diazabicyclo-[5.4.0]undec-7-en-Zugabe nicht mehr vollständig ab.
$R_f$ = 0,28 (Essigsäureethylester / Petrolether 1:4)

Beispiel XIV

(1 RS)-2,2-Dimethoxy-1-hydroxymethylcyclohexan

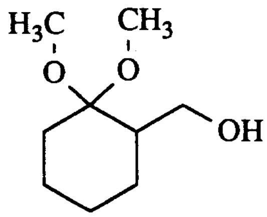

Unter Feuchtigkeitsausschluß und unter Schutzgas tropft man zu einer Suspension von 158 mg (4,16 mmol) Lithiumaluminiumhydrid in 6 ml abs. Ether eine Lösung von 1,45 g (6,93 mmol) (1 RS)-2,2-Dimethoxycyclohexan-1-carbonsäureethylester in 2 ml Ether so zu, daß der Ether mäßig siedet. Man erhitzt 1 h unter Rückfluß und rührt anschließend 15 h bei Raumtemperatur. Da die Umsetzung laut DC-Kontrolle noch nicht vollständig ist, gibt man nochmals 88 mg (2,32 mmol) Lithiumaluminiumhydrid zum Reaktionsgemisch und erhitzt 4 h unter Rückfluß. Nach beendeter Reaktion (DC-Kontrolle, nach 4 h) hydrolysiert man durch Zugabe von 252 mg 15 %iger wäßriger Natronlauge und 1,18 g Wasser. Es wird 10 min unter Rückfluß erhitzt und die überstehende Lösung vom Niederschlag abdekantiert, wobei dieser mehrmals mit insgesamt ca. 80 ml Ether gewaschen wird. Man trocknet die vereinigten Etherphasen über Natriumsulfat, filtriert und dampft das Lösemittel im Vakuum ab. Der Rückstand wird an 65 g Kieselgel (32 - 64 µm) in Essigsäureethylester / Petrolether 1:5 mit 0,7% Triethylamin chromatographiert (Flashchromatographie). Man erhält 886 mg (74%) einer farblosen Flüssigkeit als gewünschtes Produkt.

$R_f$ = 0,25 (Essigsäureethylester / Petrolether 1:4)

In Analogie zur Vorschrift des Beispiels XIV werden die in Tabelle I aufgeführten Verbindungen hergestellt:

Tabelle I:

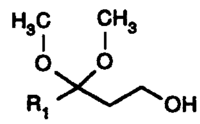

| Bsp.-Nr. | $R^1$ | $R_f$ / LM |
|---|---|---|
| XV | $H_3C$ | 0,30 (Tert.butylmethylether / Petrolether 2:1) |
| XVI | $-(CH_2)_2O-CH_2-C_6H_5$ | 0,11 (Essigester/Petrolether 1:2) |

Beispiel XVII

(5 RS)-3,3-Dimethoxy-5-ethoxyhexan-1-ol

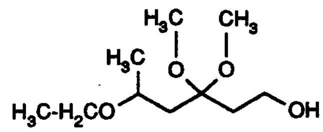

370 mg (0,83 mmol) der Verbindung aus Beispiel VII werden in 10 ml abs. Tetrahydrofuran gelöst und auf 0 °C abgekühlt (Eisbad). Innerhalb von 10 min tropft man eine Lösung von 316 mg (0,10 mmol) Tetra-n-butylammoniumfluorid / 3 $H_2O$ (vor der Reaktion 2 h im Hochvakuum bei Raumtemperatur getrocknet) in 1 ml abs. Tetrahydrofuran bei 0 °C zu und rührt 15 h bei 6 °C. Anschließend wird das Lösemittel im Vakuum abgedampft und der Rückstand durch Flashchromatographie an 40 g Kieselgel (32 - 64 µm) in Essigsäureethylester / Petrolether 1:2 mit jeweils 7 ml pro 1000 ml Laufmittel Triethylamin gereinigt. Man erhält 152 mg (89%) des gewünschten Produkts als farbloses Öl.
$R_f$ = 0,11 (Essigsäureethylester / Petrolether 1:4)
Die in Tabelle II aufgeführten Verbindungen werden in Analogie zur Vorschrift des Beispiels XVII hergestellt:

Tabelle II:

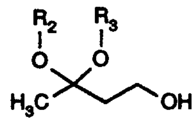

| Bsp.-Nr. | $R^2$ | $R^3$ | $R_f$ |
|---|---|---|---|
| XVIII | $-(CH_2)_2-OCH_3$ | $-(CH_2)_2-OCH_3$ | 0,23 (EE) |
| XIX | $-CH_3$ | $-(CH_2)_2-OCH_3$ | 0,06 (EE/PE 1:2) |
| XX | $-CH_3$ | $-(CH_2)_2-Br$ | 0,37 (EE/PE 1:1) |

* EE = Essigester
PE = Petrolether

Herstellungsbeispiele (allgemeine Formel (I))

Beispiel 1

(RS-P, 5 RS)-1-O-(3,3-Dimethoxy-5-ethoxy)hexyl-N,N-bis-(2-chlorethyl)phosphorsäurediamidat

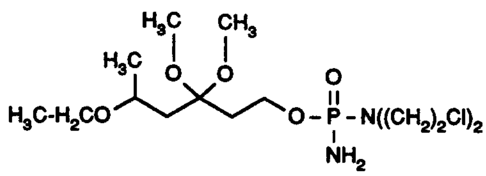

Unter Feuchtigkeitsausschluß und unter Schutzgas werden 621 mg (2,40 mmol) N,N-Bis-(2-chlorethyl)-phosporsäureamiddichlorid in 2 ml absolutem Dichlormethan sowie 1 ml Triethylamin vorgelegt. Innerhalb von 10 min tropft man eine Lösung aus 124 mg (0,60 mmol) der Verbindung aus Beispiel XVII in 1 ml abs. Dichlormethan zu und rührt 3 h bei Raumtemperatur. Nach beendeter Umsetzung (DC-Kontrolle) verdünnt man mit 10 ml Dichlormethan und leitet 45 min einen kräftigen, trockenen Ammoniakstrom durch die Reaktionsmischung, wobei verdampftes Lösemittel ersetzt wird. Die Reaktionsmischung wird anschließend zweimal über je ca. 20 g Celite (Ether) filtriert, wobei mit ca. 150 ml Ether nachgewaschen wird. Nach Abdampfen des Lösemittels im Vakuum wird der Rückstand durch Flashchromatographie an 30 g Kieselgel (32 - 64 µm) in Essigsäureethylester mit 0,7% Triethylamin gereinigt. Man erhält 125 mg (51%) eines gelben Öls als Produkt.

$R_f$ = 0,15 (Essigsäureethylester)

In Analogie zur Vorschrift des Beispiels 1 werden die in Tabelle 1 aufgeführten Verbindungen hergestellt:

Tabelle 1:

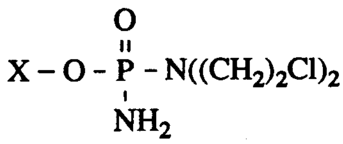

| Bsp.-Nr. | X | $R_f$ / LM |
|---|---|---|
| 2 | 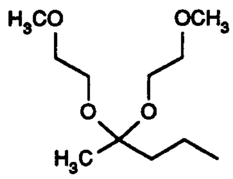 | 0,20 (Aceton/PE 1:1) |
| 3 | 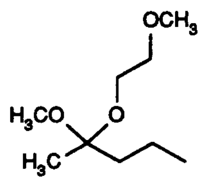 | 0,13 (tert.Butylmethylether/ Hexan/Ethanol 15:10:1) |
| 4 | 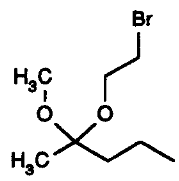 | 0,10 (tert.Butylmethylether/ Hexan/Ethanol 15:10:1) |
| 5 | 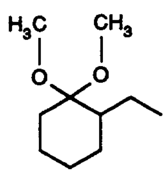 | 0,39 (EE) |
| 6 | 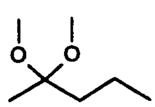 | 0,14 (Chloroform/tert.Butyl-methylether/Methanol 15:8:1) |

Beispiel 7

(RS-P)-1-O-(5-Benzyloxy-3,3-dimethoxy)pentyl-N,N-bis-(2-chlorethyl)phosphorsäurediamidat

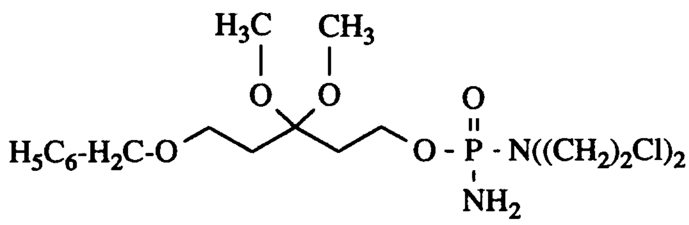

Unter Feuchtigkeitsausschluß und Schutzgas werden 2,96 g (11,4 mmol) N,N-Bis-(2-chlorethyl)-phosphorsäureamiddichlorid in 4 ml wasserfreiem Dichlormethan gelöst und bei Raumtemperatur unter Rühren mit 2 ml Triethylamin versetzt. Anschließend spritzt man unter Eiskühlung 302 µl (3,88 µmol) N-Methylimidazol langsam zu, rührt 10 min bei Raumtemperatur und tropft innerhalb von 45 min eine Lösung von 802 mg (3,15 mmol) 5-Benzyloxy-3,3-dimethoxypentan-1-ol in 2 ml wasserfreiem Dichlormethan zu. Nach 15 stündigem Rühren bei 20 °C (DC-Kontrolle) verdünnt man mit 15 ml Dichlormethan und leitet ca. 45 min einen kräftigen, trockenen Ammoniakstrom durch die Reaktionsmischung, wobei man verdampftes Lösemittel ersetzt. Nach beendeter Gaseinleitung filtriert man über 20 g Celite (Ether) und wäscht mit 200 ml Ether nach. Das Lösemittel wird im Vakuum entfernt und der Rückstand an 120 g Kieselgel (32 - 64 µm) durch Flashchromatographie gereinigt (Essigsäureethylester mit 0,7% Triethylamin). Man erhält 977 mg (68%) Produkt als gelbes, viskoses Öl.

$R_f$ = 0,14 (Essigsäureethylester / Petrolether 5:1)

Beispiel 8

(RS-P)-1-O-(3,3-Dimethoxy-5-hydroxy)pentyl-N,N-bis-(2-chlorethyl)phosphorsäurediamidat

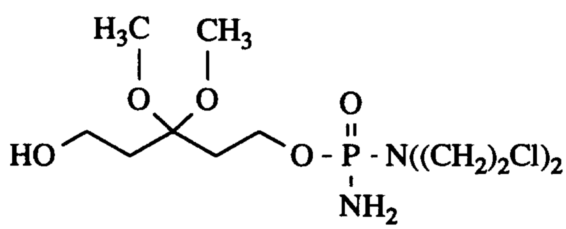

146 mg Palladium auf Aktivkohle (10%ige Belegung) und 117 mg Palladium auf Calciumcarbonat (10%ige Belegung) werden in 10 ml absolutem Methanol suspendiert und mit einer Lösung von 565 mg (1,24 mmol) der Verbindung aus Beispiel 7 in 5 ml abs. Methanol versetzt. Man hydriert 1 h bei Raumtemperatur und filtriert das Reaktionsgemisch über ca. 10 g Celite (Essigsäureethylester). Abdampfen des Lösemittels im Vakuum und Flashchromatographie an 40 g Kieselgel (32-64 µm) in Essigsäureethylester / Methanol 9:1 mit 0,7% Triethylamin liefern 398 mg (88%) Produkt.

$R_f$ = 0,25 (Essigsäureethylester / Methanol 9:1)

**Patentansprüche**

**1.** N,N-Bis-(2-Chlorethyl)phosphorsäurediamidate der allgemeinen Formel (I)

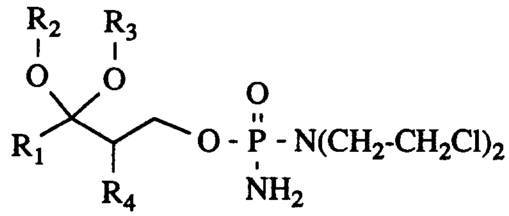

(I)

in welcher

$R^1$ für einen Zuckerrest der Formel

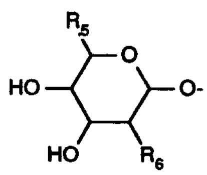

oder

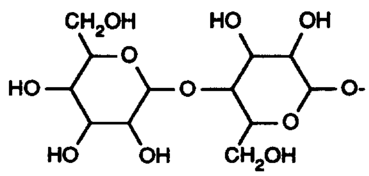

in der

D- oder L-Form steht,

worin

$R^5$ Methyl oder die $-CH_2OH$-Gruppe bedeutet

und

$R^6$ die OH-Gruppe oder einen Rest der Formel $-NR^7R^8$ bedeutet,

worin

$R^7$ und $R^8$ gleich oder verschieden sind und Wasserstoff oder eine Aminoschutzgruppe bedeuten,

wobei die Hydroxygruppen der Zuckerreste gegebenenfalls geschützt sind,

oder

für geradkettiges oder verzweigtes Alkyl mit bis zu 8 Kohlenstoffatomen steht, das gegebenenfalls bis zu 2-fach gleich oder verschieden durch Hydroxy, geschütztes Hydroxy oder geradkettiges oder verzweigtes Alkoxy mit bis zu 6 Kohlenstoffatomen, Benzyl oder Benzyloxy oder einen der oben aufgeführten Zuckerreste der Formeln

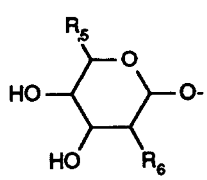

oder

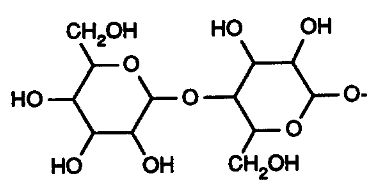

in der

D- oder L-Form substituiert ist,

worin

$R^5$ und $R^6$ die oben angegebene Bedeutung haben,

$R^2$ und $R^3$ gleich oder verschieden sind und

für Aryl mit 6 bis 10 Kohlenstoffatomen stehen

für geradkettiges oder verzweigtes Alkyl mit bis zu 8 Kohlenstoffatomen stehen, das gegebenenfalls durch Halogen, Hydroxy, geschütztes Hydroxy, geradkettiges oder verzweigtes Alkoxy mit bis zu 8 Kohlenstoffatomen oder durch eine Gruppe der Formel $-NR^9R^{10}$ substituiert ist,

worin

$R^9$ und $R^{10}$ gleich oder verschieden sind und Wasserstoff, geradkettiges oder verzweigtes Alkyl mit bis zu 6 Kohlenstoffatomen oder eine Gruppe der Formel -CO-$R^{11}$ bedeuten,

worin

$R^{11}$ geradkettiges oder verzweigtes Alkyl mit bis zu 6 Kohlenstoffatomen oder Trifluormethyl bedeutet,

$R^4$ für Wasserstoff oder für geradkettiges oder verzweigtes Alkyl mit bis zu 8 Kohlenstoffatomen steht,

oder

$R^1$ und $R^4$ gemeinsam einen 5- bis 7-gliedrigen, gesättigten Carbocyclus bilden.

**2.** Verbindungen der allgemeinen Formel (I), gemäß Anspruch 1 in welcher

$R^1$ für einen Zuckerrest der Formel

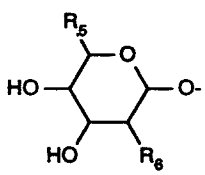

oder

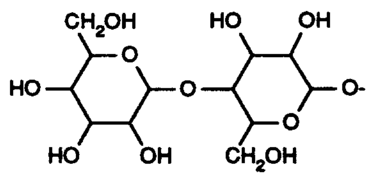

in der

D- oder L-Form steht,

worin

$R^5$ Methyl oder die -$CH_2OH$-Gruppe bedeutet

und

$R^6$ die OH-Gruppe oder einen Rest der Formel -$NR^7R^8$ bedeutet,

worin

$R^7$ und $R^8$ gleich oder verschieden sind und Wasserstoff oder Acetyl bedeuten,

wobei die Hydroxygruppen der Zuckerreste gegebenenfalls durch Acetyl, Benzyl oder Methylbenzoyl geschützt sind,

für geradkettiges oder verzweigtes Alkyl mit bis zu 6 Kohlenstoffatomen steht, das gegebenenfalls durch Hydroxy oder durch geschütztes Hydroxy, geradkettiges oder verzweigtes Alkoxy mit bis zu 4 Kohlenstoffatomen, Benzyl oder durch einen der oben aufgeführten Zuckerreste der Formel

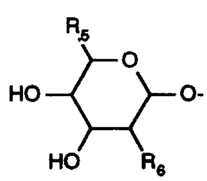

oder

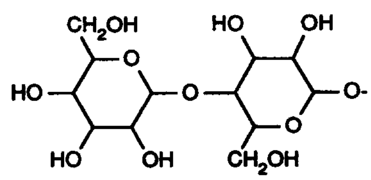

in der D- oder L-Form substituiert ist,

worin

$R^5$ und $R^6$ die oben angegebene Bedeutung haben,

$R^2$ und $R^3$ gleich oder verschieden sind und für Phenyl oder geradkettiges oder verzweigtes Alkyl mit bis zu 6 Kohlenstoffatomen stehen, das gegebenenfalls durch Fluor, Chlor, Brom, Hydroxy, geradkettiges oder verzweigtes Alkoxy mit bis zu 6 Kohlenstoffatomen oder durch eine Gruppe der Formel -$NR^9R^{10}$ substituiert ist,

worin

$R^9$ und $R^{10}$ gleich oder verschieden sind und Wasserstoff, geradkettiges oder verzweigtes

Alkyl mit bis zu 4 Kohlenstoffatomen oder eine Gruppe der Formel $-CO-R^{11}$ bedeuten,
worin

$R^{11}$ geradkettiges oder verzweigtes Alkyl mit bis zu 4 Kohlenstoffatomen oder Trifluormethyl bedeutet,

$R^4$ für Wasserstoff oder für geradkettiges oder verzweigtes Alkyl mit bis zu 6 Kohlenstoffatomen steht,

oder

$R^1$ und $R^4$ gemeinsam einen Cyclopentyl- oder Cyclohexylring bilden.

**3.** Verbindungen der allgemeinen Formel (I), gemäß Anspruch 1
in welcher

$R^1$ für einen Zuckerrest der Formel

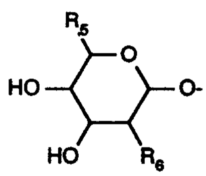

oder

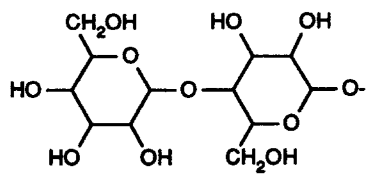

in der

D- oder L-Form steht,
worin

$R^5$ die $-CH_2OH$-Gruppe bedeutet
und

$R^6$ die OH-Gruppe oder einen Rest der Formel $-NR^7R^8$ bedeutet,
worin

$R^7$ und $R^8$ gleich oder verschieden sind und Wasserstoff oder Acetyl bedeuten,
wobei die Hydroxygruppen der Zuckerreste gegebenenfalls durch Acetyl, Benzyl oder Methylbenzoyl geschützt sind,
für geradkettiges oder verzweigtes Alkyl mit bis zu 4 Kohlenstoffatomen steht, das gegebenenfalls durch Hydroxy, Methoxy, Ethoxy, Propoxy, Isopropoxy, Benzyl, Benzyloxy oder durch einen der oben aufgeführten Zuckerreste der Formel

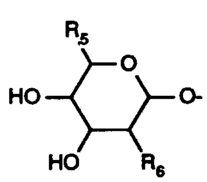

oder

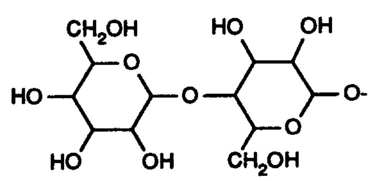

in der

D- oder L-Form substituiert ist,
worin

$R^5$ und $R^6$ die oben angegebene Bedeutung haben,

$R^2$ und $R^3$ gleich oder verschieden sind und für Phenyl oder geradkettiges oder verzweigtes Alkyl mit bis zu 4 Kohlenstoffatomen stehen, das gegebenenfalls durch Fluor, Chlor, Brom, Hydroxy, geradkettiges oder verzweigtes Alkoxy mit bis zu 4 Kohlenstoffatomen oder durch eine Gruppe der Formel $-NR^9R^{10}$ substituiert ist,
worin

$R^9$ und $R^{10}$ gleich oder verschieden sind und Wasserstoff, Methoxy, Ethyl, Propyl, Isopropyl oder eine Gruppe der Formel $-CO-R^{11}$ bedeuten,
worin

$R^{11}$ Methyl, Ethyl oder Trifluormethyl bedeutet

$R^4$ für Wasserstoff oder für geradkettiges oder verzweigtes Alkyl mit bis zu 4 Kohlenstoffatomen steht,

oder

$R^1$ und $R^4$ gemeinsam einen Cyclopentyl- oder Cyclohexylring bilden.

**4.** Verfahren zur Herstellung der erfindungsgemäßen Verbindungen der allgemeinen Formel (I) gemäß Anspruch 1, dadurch gekennzeichnet, daß man Verbindungen der allgemeinen Formel (II)

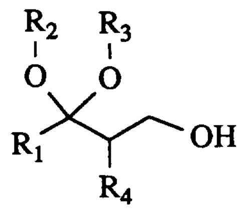

(II)

in welcher

$R^1$, $R^2$, $R^3$ und $R^4$ die oben angegebene Bedeutung haben,

zunächst mit N,N-Bis-(2-Chlorethyl)phosphorsäureamiddichlorid der Formel (III)

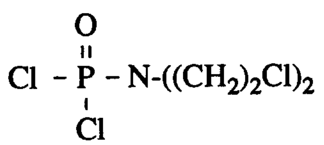

(III)

in inerten Lösemitteln, in Anwesenheit einer Base und eines Hilfsstoffes, umsetzt, anschließend eine Ammonolyse durchführt, und im Fall, daß $R^1$ und/oder einer der oben aufgeführten Zuckerreste eine Schutzgruppe trägt, diese nach üblicher Methode abspaltet.

**5.** Arzneimittel enthaltend eine oder mehrere Verbindungen der Ansprüche 1 bis 3.

Europäisches Patentamt

# EUROPÄISCHER RECHERCHENBERICHT

Nummer der Anmeldung
EP 93 10 0650

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| --- | --- | --- | --- |
| Y | WO-A-9 010 636 (BOARD OF REGENTS, THE UNIVERSITY OF TEXAS SYSTEM)<br>* das ganze Dokument *<br>--- | 1,5 | C07F9/24<br>A61K31/66<br>C07H13/04<br>A61K31/70 |
| Y | CHEMICAL ABSTRACTS, vol. 78, no. 11, 19. März 1973, Columbus, Ohio, US;<br>abstract no. 71418,<br>Seite 413 ;<br>* Zusammenfassung *<br>& JP-A-47 038 928 (SHIONOGI AND CO. LTD.)<br>6. Dezember 1972<br>--- | 1,5 | |
| Y | EP-A-0 415 198 (BAYER AG)<br>* das ganze Dokument *<br>----- | 1,5 | |
| | | | RECHERCHIERTE SACHGEBIETE (Int. Cl.5) |
| | | | C07F<br>C07H<br>A61K |

Der vorliegende Recherchenbericht wurde für alle Patentansprüche erstellt

| Recherchenort | Abschlußdatum der Recherche | Prüfer |
| --- | --- | --- |
| DEN HAAG | 06 MAI 1993 | BESLIER L.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)